# FASCICULE DE BREVET EUROPEEN

(11) **EP 3 016 602 B1**
(45) Date de publication et mention de la délivrance du brevet: **27.12.2017**
(21) Numéro de dépôt: 14733269.6
(22) Date de dépôt: 05.06.2014
(51) Int. Cl.: A61B 17/34, A61B 17/22, A61B 10/02

(54) **TROCART PERFORANT**
PERFORIERENDER TROKAR
PERFORATING TROCAR

(30) Priorité: 01.07.2013 FR 1356391
(43) Date de publication de la demande: 11.05.2016
(73) Titulaire: Fumex, Laurent, Madison, CT 06443 (US); Masseglia, Thierry, 83130 La Garde (FR)
(72) Inventeur: Fumex, Laurent, Madison, CT 06443 (US); Masseglia, Thierry, 83130 La Garde (FR)
(74) Mandataire: Bradley, Adrian
(86) Numéro de dépôt international: PCT/FR2014/051346
(87) Numéro de publication internationale: WO 2015/001211

(56) Documents cités:
- US-A1- 2003 225 411
- US-A1- 2009 204 024
- US-B1- 6 575 919
- US-B2- 7 850 620

## Description

La présente invention concerne un dispositif utilisable en chirurgie et en radiologie interventionnelle, et plus particulièrement un trocart perforant utilisable notamment dans le domaine des procédures percutanées de la biopsie osseuse ou de moelle, de la vertébroplastie, de la cimentoplastie des régions squelettiques, et plus généralement du traitement des lésions osseuses.

On connaît divers types de trocarts perforants qui sont des instruments chirurgicaux utilisés pour forer l'os de façon à atteindre une zone où la biopsie osseuse doit être effectuée. Ces trocarts sont composés d'un tube externe creux, dont l'extrémité est plus ou moins coupante, et d'une tige, dont l'extrémité est affûtée de façon à perforer l'os, coulissant dans le tube.

Ainsi, la demande de brevet WO 2006/061514 décrit un trocart destiné à la biopsie osseuse comprenant un tube externe, dont l'extrémité distale est divisée en deux segments à bord coupant hélicoïdal, dans lequel coulisse une tige affûtée. Ce type d'instrument est utilisé manuellement au moyen d'une poignée.

Le brevet US 7850620 décrit un trocart destiné à la biopsie de moelle osseuse composé d'un tube externe, dont l'extrémité distale comporte un affûtage traditionnel pour ce type d'instrument, associé à une tige affûtée de façon traditionnelle. Cet instrument est utilisé en l'accouplant à une perceuse.

Les demandes de brevet US 2003/225411 A1 (le préambule de la revendication 1 est basé sur ce document) et US 2009/0204024 A1 concernent des trocarts destinés à la biopsie de moelle osseuse. Ces trocarts comportent chacun une tige ayant une encoche permettant l'évacuation de morceaux d'os et de tissu. Le brevet US 6,575,919 B1 décrit un trocart ayant une ouverture intérieure pour permettre le passage d'une aiguille.

Les trocarts perforants connus permettent de forer l'os mais ne peuvent pas être guidés sur broche en même temps. Cette lacune présente deux inconvénients majeurs : l'absence de précision au moment de l'abord osseux et le risque d'accident. Or, les procédures percutanées sont essentiellement effectuées sous imagerie et donc à partir d'images. Le point d'entrée et la trajectoire peuvent ainsi être visualisés et définis à l'avance. L'importance de la précision du point d'entrée se comprend parfaitement en cas de petites lésions osseuses car celles-ci ont des diamètres ne dépassant parfois pas le millimètre ; la difficulté est naturellement accrue lorsqu'il s'agit d'atteindre une lésion trouvant son siège dans un os profond. Ainsi il n'est pas rare chez un patient obèse d'avoir à traverser 150mm à 200mm de parties molles avant d'atteindre la corticale osseuse. Maintenir dans de telles conditions au millimètre près la trajectoire du trocart dont le diamètre est d'environ 2mm est particulièrement difficile. De plus, il y a des risques liés à une introduction directe d'un trocart perforant lorsque cet os est situé dans une zone dense comprenant des organes, des veines et des nerfs. Confronté à de telles situations, le praticien acceptera de multiplier les gestes et placera d'abord manuellement une broche rigide fine et peu invasive, laquelle servira de guide aux divers trocarts et instruments coaxiaux pour la suite de la procédure opératoire.

On connait également des mèches perforées, utilisées en orthopédie, qui permettent de forer l'os en étant guidées sur broche. Cependant, ces mèches ne sont pas ou peu employées dans le cadre d'une procédure percutanée et encore moins lorsque celle-ci est effectuée manuellement. L'utilisation de ces mèches exige que la broche soit d'abord fichée dans l'os au moyen d'une perceuse ; en effet, ces mèches n'ont pas de pointe distale étant perforées de part en part afin de coulisser sur la broche. Elles risquent donc, en l'absence de broche fichée, de glisser sur l'os et de dévier du point d'entrée défini par le praticien.

La présente invention a pour objet un dispositif, notamment pour la chirurgie et la radiologie interventionnelle, procurant une bonne efficacité de perforation tout en étant parfaitement adapté pour être guidé sur une broche rigide jusqu'au contact osseux. L'invention a encore pour objet un dispositif permettant, au choix du praticien, de débuter une procédure opératoire manuellement puis de la terminer au moyen d'un entrainement automatique en rotation, telle qu'une perceuse, si la dureté de l'os le nécessite, et ce sans perte du point d'entrée dans l'os.

Le dispositif selon la présente invention, du type trocart perforant, comprend une gaine externe comportant un tube rigide et un mandrin comportant une tige. La tige est adaptée pour coulisser dans la gaine externe et comporte à son extrémité distale une pointe perforante. La tige comporte en outre une rainure longitudinale sur la surface de la tige s'étendant de l'extrémité distale à l'extrémité proximale de la tige pour permettre le coulissage du dispositif sur une broche guide.

Selon un mode de réalisation de l'invention, la rainure est en forme de V.

Préférentiellement, la pointe perforante est formée par un affûtage à biseaux de l'extrémité distale de la tige. Selon une variante préférée, l'extrémité distale de la tige comprend trois biseaux dont l'un est moins incliné que les deux autres par rapport à l'axe de la tige, et une pointe perforante centrée par rapport à l'axe de la tige.

Avantageusement, l'extrémité distale de la tige comprend au moins une arête de coupe s'étendant depuis la pointe perforante jusqu'aux bords de la tige. Selon un mode de réalisation, cette arête de coupe est située sur une des faces de la rainure. Préférentiellement, cette arête de coupe est plus inclinée qu'une autre arête de coupe par rapport à l'axe de la tige.

L'affutage pyramidal, à biseaux, avec l'arête de coupe dont l'angle est le plus important par rapport à l'axe de la tige située sur une des faces de la rainure longitudinale, permet d'avoir une meilleure pénétration dans l'os. En effet, des essais pratiqués sur un os à moelle de boeuf ont mis en évidence que la pointe ainsi formée pénètre jusqu'à 5 fois plus profondément qu'une pointe triangulaire traditionnelle.

Suivant une caractéristique de l'invention, l'extrémité distale du tube comprend au moins deux segments à bord coupant de forme hélicoïdale.

De manière avantageuse, la gaine externe comprend en outre un raccord à l'extrémité proximale du tube, et le mandrin comprend en outre un bouchon à l'extrémité proximale de la tige. Le bouchon est adapté pour recevoir le raccord afin de former un ensemble solidarisé. Le raccord est perforé pour permettre le coulissage de la tige, et le bouchon est perforé pour permettre le coulissage de la broche guide.

Selon des modes de réalisation, l'ensemble formé de la gaine externe et du mandrin est monté dans une poignée ou dans un moyen automatique d'entrainement en rotation. Par exemple, le moyen automatique d'entrainement en rotation peut être une perceuse ayant un embout dans lequel est monté l'ensemble.

Avantageusement, le moyen automatique d'entrainement peut être pourvu d'une enveloppe protectrice. L'enveloppe protectrice peut, par exemple, être attachée à l'embout d'une perceuse au moyen d'un raccord qui est simultanément adapté à recevoir la gaine externe du trocart perforant.

À titre d'exemple, dans le cas d'une lésion osseuse siégeant sous une corticale dense, le praticien, après avoir procédé à une anesthésie locale suivant les techniques classiques, introduit la broche guide parallèlement ou au travers de l'aiguille anesthésique en place jusqu'à venir au contact de l'os. L'aiguille anesthésique est ensuite enlevée tout en laissant la broche guide en place. Le dispositif selon l'invention, équipé d'une poignée amovible, est introduit au travers des tissus jusqu'au contact de l'os tout en étant guidé sur broche. La broche est enlevée une fois la surface de la corticale atteinte, puis le praticien fore l'os en tournant manuellement le trocart sans perte du point d'entrée. Dans 80% à 90% des cas le forage se fera manuellement, mais si la paroi osseuse est très dure, le praticien peut enlever la poignée amovible et raccorder le trocart à une perceuse pour terminer la procédure opératoire.

La simplicité de la structure du dispositif de l'invention permet de proposer à l'utilisateur, en fonction de la profondeur ou de la localisation de la lésion, un instrument unique permettant au choix une voie directe ou bien guidée sur broche, et dont le forage peut être manuel ou à la perceuse, ou bien encore commencé manuellement pour être terminé à la perceuse. La combinaison d'un forage manuel avec un forage à la perceuse permet de bénéficier à la fois d'une grande précision de l'acte opératoire et d'une grande puissance quelle que soit la dureté de l'os.

D'autres caractéristiques et avantages de la présente invention apparaîtront dans la description suivante, relative à une forme préférentielle de réalisation, en référence aux dessins annexés, qui représentent :
- Figure 1 : vue en perspective d'un dispositif selon l'invention monté dans une poignée ;
- Figures 2 à 3 : vues en perspective d'un tube du dispositif selon l'invention monté dans un raccord, formant une gaine externe ;
- Figures 4 à 5 : vues en perspective d'une tige rainurée du dispositif selon l'invention montée dans un bouchon, formant un mandrin ;
- Figures 6 à 9 : vues d'un exemple de l'extrémité distale de la tige rainurée ;
- Figures 10 et 11 : vues d'un exemple de l'extrémité distale de la tige pourvue d'un trou ;
- Figure 12 : vue en perspective d'une poignée apte à recevoir l'ensemble formé de la gaine et du mandrin du dispositif selon l'invention ; et
- Figure 13 : vue en perspective d'une perceuse apte à recevoir l'ensemble formé de la gaine et du mandrin du dispositif selon l'invention.

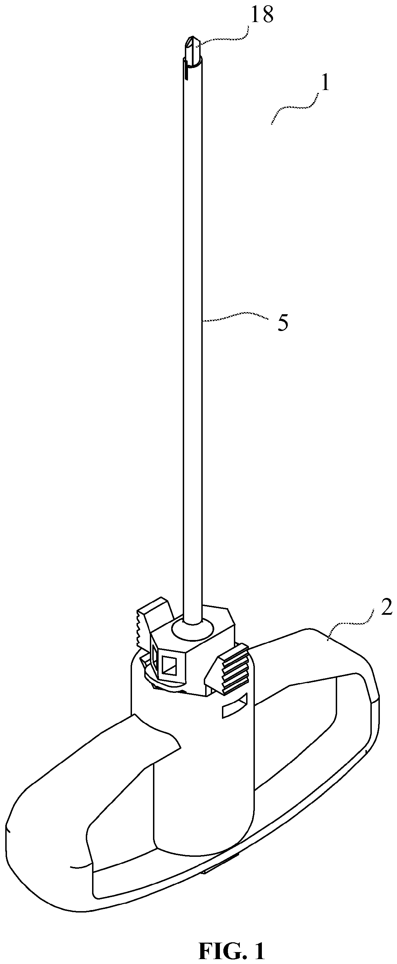

Le trocart 1, représentée sur la Figure 1, est composé d'une gaine externe 5 dans laquelle est monté un mandrin (dont seulement l'extrémité distale de la tige 18 est visible sur la Figure 1), et d'une poignée 2 dans laquelle l'ensemble gaine-mandrin est inséré.

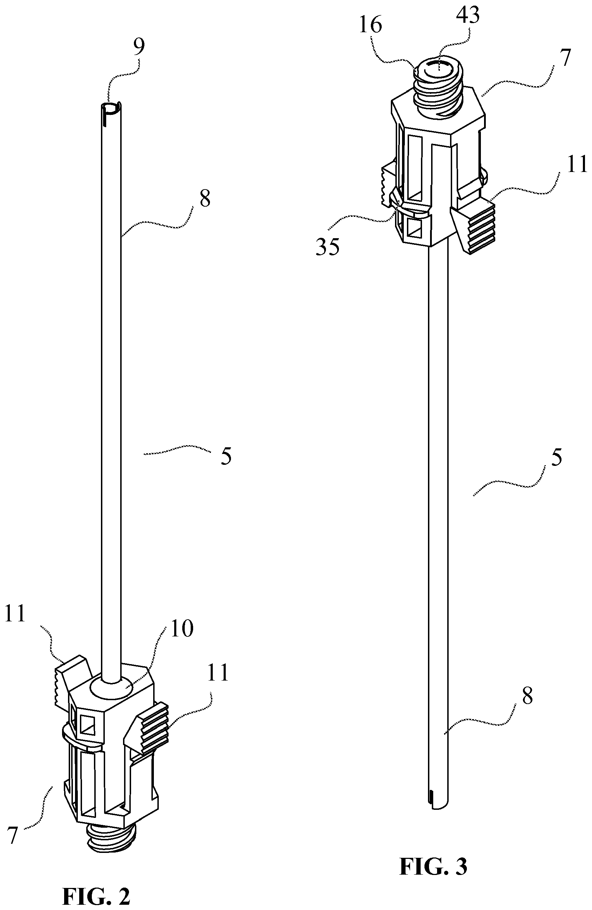

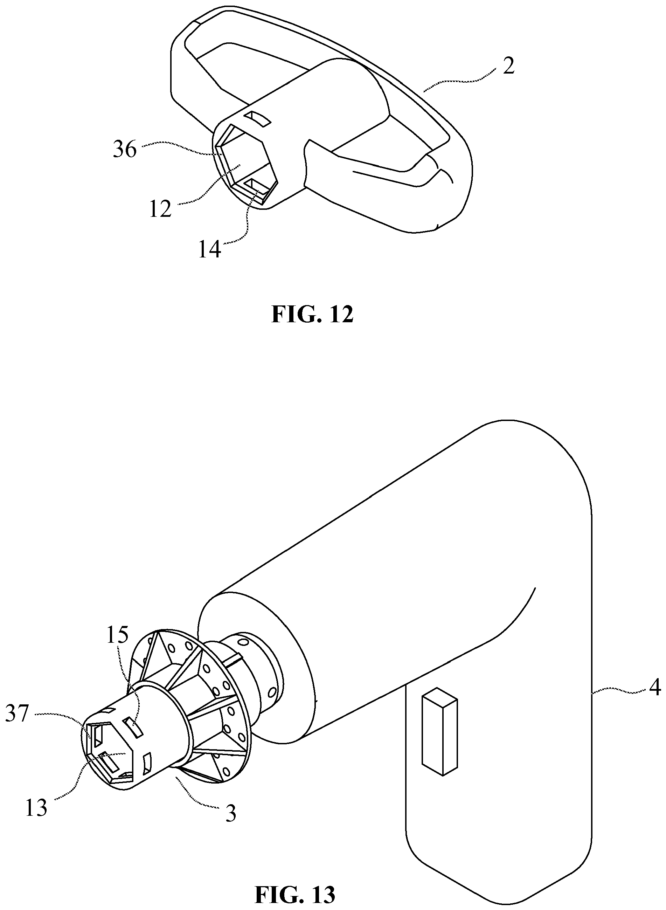

La gaine externe 5, représentée sur les Figures 2 et 3, comporte un raccord 7 dans lequel est logé le tube affûté 8. Le tube 8 comporte une extrémité distale 9 divisée en deux segments à bord coupant hélicoïdal tel que décrit dans la demande WO 2006/061514. Le raccord 7 est de forme hexagonale coopérant avec des cavités hexagonales 12 de la poignée 2 et des cavités hexagonales 13 de l'embout 3 d'une perceuse 4 (voir également les Figures 12 et 13). Le raccord 7 comporte un perçage 10, dans lequel le tube 8 est logé, et deux parties flexibles 11 s'encliquetant dans des évidements 14 de la poignée 2 ou dans des évidements 15 de l'embout 3 de la perceuse 4.

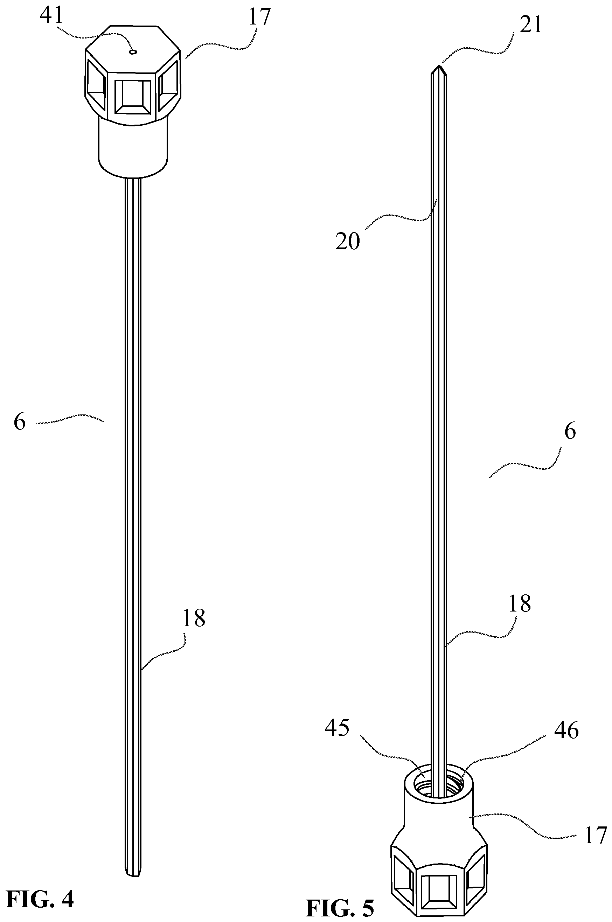

Le mandrin 6, représenté sur les Figures 4 et 5, comprend une tige rainurée 18 logée dans un bouchon 17. La tige rainurée 18 comporte une rainure longitudinale 20 en forme de V et une extrémité distale de forme pyramidale. Le bouchon 17 est de forme hexagonale coopérant avec les cavités hexagonales 12 de la poignée 2 et les cavités hexagonales 13 de l'embout 3 de la perceuse 4. Le bouchon 17 comporte un perçage 45 dans lequel la tige rainurée 18 est logée et un perçage 41 laissant passer la broche guide.

Le raccord 7 de la gaine externe 5 est entièrement perforé 43 de façon à permettre le coulissage de la tige 18. L'extrémité 16 de la gaine externe 5 de type embout Luer permet le vissage du bouchon 17. Le bouchon 17 comporte un filetage 46 coopérant avec le filetage 16 du raccord 7 permettant son vissage afin d'assembler la gaine externe 5 et le mandrin 6.

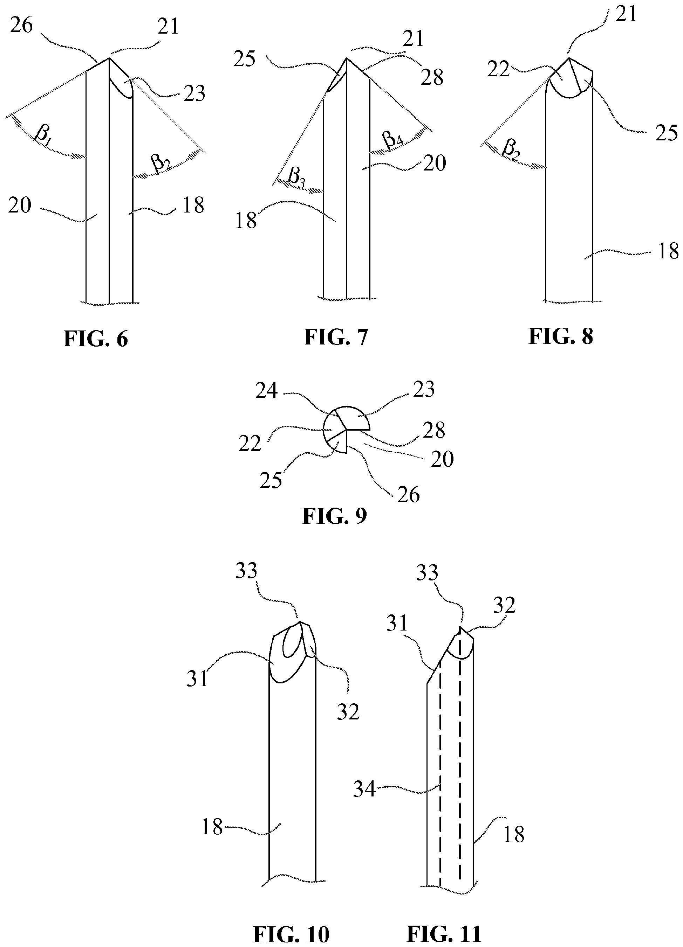

Les Figures 6 à 9 représentent des vues d'un exemple de l'extrémité distale de la tige 18. L'extrémité distale de la tige 18 comprend une pointe 21 ayant un affûtage pyramidal à trois biseaux. Deux des biseaux 22, 23 ont la même inclinaison (β₃), préférentiellement β₃ = 30° par rapport à l'axe de la tige 18, et s'étendent sur environ 120° de la section de la tige 18, générant une arête de coupe 24. Le troisième biseau 25, incliné préférentiellement suivant β₁ et à 40° par rapport à l'axe de la tige 18, génère une deuxième arête de coupe 26. La deuxième arête de coupe 26 est située sur l'une des faces de la rainure 20 et a une inclinaison β₁ plus importante que l'inclinaison β₂ de l'arête 24 par rapport à l'axe de la tige 18. Ceci permet d'avoir une meilleure coupe de l'os car la deuxième arête de coupe 26 bénéficie d'un angle d'attaque plus important, généré par l'une des faces de la rainure 20. L'inclinaison β₁ de l'arête de coupe 26 est plus importante que l'inclinaison β₂ de l'arête de coupe 24 et que l'inclinaison β₄ de l'autre arête de coupe 28 située sur l'autre face de la rainure longitudinale. L'intersection des trois biseaux génère la pointe 21 permettant au mandrin 6 de ne pas glisser sur l'os.

Les vues des Figures 10 et 11 montrent une extrémité distale de la tige 18 selon une variante. La tige 18 comporte, centré sur son axe, un trou 34 permettant le guidage sur broche. L'extrémité distale de la tige 18 comprend un affûtage pyramidal à trois biseaux de même inclinaison dont un biseau 31 est plus grand que les deux autres biseaux 32 de façon à générer une pointe 33 situé sur le bord du trou 34.

La vue en perspective de la Figure 12 montre la poignée 2 comportant une cavité hexagonale 12 dans laquelle se monte la gaine externe 5 équipée du mandrin 6. Les parties flexibles 11 du raccord 7 s'encliquettent dans les évidements 14 pourvus à cet effet.

La vue en perspective de la Figure 13 montre une perceuse 4 sur laquelle est monté l'embout 3 comportant une cavité hexagonale 13, permettant le montage de la gaine externe 5 équipée du mandrin 6, et des évidements 15 dans lesquels s'encliquettent les parties flexibles 11 du raccord 7.

En se référant aux Figures 2, 3, 12 et 13, le chanfrein 35 du raccord 7 de la gaine externe 5 coopère avec les chanfreins 36 de la poignée 2 et les chanfreins 37 de l'embout 3 de la perceuse 4 de façon à réduire les jeux lors du forage de l'os.

## Revendications

1. Dispositif, notamment pour la chirurgie et la radiologie interventionnelle, comprenant :
- une gaine externe (5) comportant un tube rigide (8) ; et
- un mandrin (6) comportant une tige (18), la tige (18) étant adaptée pour coulisser dans la gaine externe (5) et comportant une rainure longitudinale (20) sur la surface de la tige (18),
**caractérisé en ce que** la rainure longitudinale (20) s'étend de l'extrémité distale à l'extrémité proximale de la tige (18) pour permettre le coulissage du dispositif sur une broche guide, la tige (18) comportant à son extrémité distale une pointe perforante (21) formée par un affûtage pyramidal à biseaux (22, 23, 25) de l'extrémité distale de la tige (18), l'extrémité distale de la tige (18) comprenant des arêtes de coupe (24, 26, 28) s'étendant depuis la pointe perforante (21) jusqu'aux bords de la tige (18), une première arête de coupe (26) étant située sur une des faces de la rainure (20) et étant plus inclinée qu'une deuxième arête de coupe (24) par rapport à l'axe de la tige (18).

2. Dispositif selon la revendication 1, dans lequel la rainure (20) est en forme de V.

3. Dispositif selon la revendication 1 ou 2, dans lequel l'extrémité distale du tube (8) comprend au moins deux segments à bord coupant de forme hélicoïdale.

4. Dispositif selon l'une des revendications précédentes, dans lequel :
- la gaine externe (5) comprend en outre un raccord (7) à l'extrémité proximale du tube (8), et
- le mandrin comprend en outre un bouchon (17) à l'extrémité proximale de la tige (18),
le bouchon (17) étant adapté pour recevoir le raccord (7) afin de former un ensemble solidarisé,
dans lequel le raccord (7) est perforé (43) pour permettre le coulissage de la tige (18), et le bouchon (17) est perforé (41) pour permettre le coulissage de la broche guide.

5. Dispositif selon l'une des revendications précédentes, dans lequel l'ensemble formé de la gaine externe (5) et du mandrin (6) est monté dans une poignée (2) ou dans un moyen automatique d'entrainement en rotation (4).

## Patentansprüche

1. Gerät, insbesondere für Operation und interventionelle Radiologie, umfassend:
- eine äußere Hülse (5), die eine steife Röhre (8) aufweist; und
- eine Welle (6) die einen Stab (18) aufweist, wobei der Stab (18) geeignet zum Gleiten in der äußeren Hülse (5) ist und eine Längsnut (20) auf der Oberfläche des Stabs (18) aufweist, **dadurch gekennzeichnet, dass** die Längsnut (20) sich von dem distalen Ende zu dem proximalen Ende des Stabs (18) erstreckt, um dem Gerät zu gestatten auf einem Führungsstift zu gleiten, wobei der Stab (18) auf seinem distalen Ende eine Perforierspitze (21) aufweist, die durch eine Pyramidenspitze mit Schrägen (22, 23, 25) des distalen Endes des Stabs (18) gebildet wird, wobei das distale Ende des Stabs (18) Schneidegrate (24, 26, 28) umfasst, die sich von der Perforierspitze (21) zu den Kanten des Stabs (18) erstrecken, wobei ein erster Schneidegrat (26) auf einer Seite der Nut (20) angeordnet ist und mehr geneigt ist in Bezug auf die Achse des Stabs (18) als ein zweiter Schneidegrat (24).

2. Gerät wie in Anspruch 1 beansprucht, wobei die Nut (20) V-förmig ist.

3. Gerät wie in Anspruch 1 oder 2 beansprucht, wobei das distale Ende der Röhre (8) wenigstens zwei Segmente mit einer spiralförmigen Schneidekante umfasst.

4. Gerät wie in einem der vorherigen Ansprüche beansprucht, wobei:
- die äußere Hülse (5) ferner ein Verbindungsstück (7) an dem proximalen Ende der Röhre (8) umfasst, und
- die Welle ferner einen Stopfen (17) an dem proximalen Ende des Stabs (18) umfasst, wobei der Stopfen (17) zum Aufnehmen des Verbindungsstücks (7) geeignet ist, um eine integrale Baugruppe zu bilden,
wobei das Verbindungsstück (7) perforiert ist (43), um das Gleiten des Stabs (18) zuzulassen und der Stopfen (17) perforiert ist (41), um das Gleiten des Führungsstifts zuzulassen.

5. Gerät wie in einem der vorherigen Ansprüche beansprucht, wobei die durch die äußere Hülse (5) und die Welle (6) gebildete Baugruppe in einem Griff (2) oder in einem automatisch drehenden Antriebsmittel (4) montiert ist.

## Claims

1. Device, in particular for surgery and interventional radiology, comprising:
- an outer sleeve (5) having a rigid tube (8); and
- a mandrel (6) having a rod (18), the rod (18) being suitable for sliding in the outer sleeve (5) and having a longitudinal groove (20) on the surface of the rod (18), **characterised in that** the longitudinal groove (20) extends from the distal end to the proximal end of the rod (18) in order to allow the device to slide on a guide pin, the rod (18) having, on its distal end, a perforating tip (21) formed by a pyramid point with bevels (22, 23, 25) of the distal end of the rod (18), the distal end of the rod (18) comprising cutting ridges (24, 26, 28) extending from the perforating tip (21) to the edges of the rod (18), a first cutting ridge (26) being situated on one of the faces of the groove (20) and being more inclined than a second cutting ridge (24) with respect to the axis of the rod (18).

2. Device as claimed in claim 1, wherein the groove (20) is V-shaped.

3. Device as claimed in claim 1 or 2, wherein the distal end of the tube (8) comprises at least two segments with a helical cutting edge.

4. Device as claimed in any one of the preceding claims, wherein:
- the outer sleeve (5) further comprises a connector piece (7) at the proximal end of the tube (8), and
- the mandrel further comprises a stopper (17) at the proximal end of the rod (18), the stopper (17) being suitable for receiving the connector piece (7) in order to form an integral assembly,
wherein the connector piece (7) is perforated (43) in order to permit the sliding of the rod (18), and the stopper (17) is perforated (41) in order to permit the sliding of the guide pin.

5. Device as claimed in any one of the preceding claims, wherein the assembly formed by the outer sleeve (5) and the mandrel (6) is mounted in a handle (2) or in an automatic rotational drive means (4).
